(19) **Europäisches Patentamt European Patent Office Office européen des brevets**

(11) **EP 1 801 590 B1**

(12) **EUROPEAN PATENT SPECIFICATION**

(45) Date of publication and mention of the grant of the patent:
**28.07.2010 Bulletin 2010/30**

(21) Application number: **05776096.9**

(22) Date of filing: **30.08.2005**

(51) Int Cl.:
**G01N 33/543** (2006.01)

(86) International application number:
**PCT/JP2005/015788**

(87) International publication number:
**WO 2006/025401 (09.03.2006 Gazette 2006/10)**

(54) **METHOD OF ASSAYING ANTIGEN AND REAGENT THEREFOR**

VERFAHREN ZUM TESTEN EINES ANTIGENS UND REAGENS DAFÜR

PROCÉDÉ D'ANALYSE D'ANTIGÈNE ET RÉACTIF CORRESPONDANT

(84) Designated Contracting States:
**AT BE BG CH CY CZ DE DK EE ES FI FR GB GR HU IE IS IT LI LT LU LV MC NL PL PT RO SE SI SK TR**

(30) Priority: **31.08.2004 JP 2004251456**

(43) Date of publication of application:
**27.06.2007 Bulletin 2007/26**

(73) Proprietor: **DENKA SEIKEN Co., Ltd.**
**Chuo-ku,**
**Tokyo 103-0025 (JP)**

(72) Inventors:
• **MOTODA, Shosaku,**
**Denka Seiken Co., Ltd.**
**Gosen-shi, Niiga ta 9591695 (JP)**
• **MINAKAWA, Yasunori,**
**Denka Seiken Co., Ltd.**
**Gosen-shi, Niigata 9591695 (JP)**

(74) Representative: **Kremer, Simon Mark et al**
**Mewburn Ellis LLP**
**33 Gutter Lane**
**London**
**EC2V 8AS (GB)**

(56) References cited:
EP-A- 0 898 169    JP-A- 2004 157 072
JP-B- 63 014 783    JP-B2- 3 513 075
US-A1- 2001 026 945

• **SÁNCHEZ ANGELA ET AL: "Evaluation of an improved immunoturbidimetic assay for serum C-reactive protein on a COBAS INTEGRA 400 Analyzer" CLINICAL LABORATORY, CLIN LAB PUBLICATIONS, HEIDELBERG, vol. 48, no. 5-6, 1 January 2002 (2002-01-01), pages 313-317, XP008094804 ISSN: 1433-6510**
• **EDA S ET AL: "A new method of measuring C-reactive protein, with a low limit of detection, suitable for risk assessment of coronary heart disease" SCANDINAVIAN JOURNAL OF CLINICAL AND LABORATORY INVESTIGATION.SUPPLEMENT, BLACKWELL SCIENTIFIC PUBLICATIONS, OXFORD, GB, vol. 230, 1 January 1999 (1999-01-01), pages 32-35, XP008094803 ISSN: 0085-591X**
• **EDA S ET AL: "Development of a new microparticle-enhanced turbidimetric assay for C-reactive protein with superior features in analytical sensitivity and dynamic range." JOURNAL OF CLINICAL LABORATORY ANALYSIS 1998, vol. 12, no. 3, 1998, pages 137-144, XP002490485 ISSN: 0887-8013**
• **KATANO SHINJI: "Application of serum protein test by full-automated clinical chemistry analyzer COBAS INTEGRA 400" IGAKU TO YAKUGAKU - JOURNAL OF MEDICINE, SHIZEN KAGAKUSGA, TOKYO, JP, vol. 42, no. 5, 1 January 1999 (1999-01-01), pages 781-788, XP008094844 ISSN: 0389-3898**
• **BENECKY M J ET AL: "Detection of hepatitis B surface antigen in whole blood by coupled particle light scattering (Copalis)" CLINICAL CHEMISTRY, AMERICAN ASSOCIATION FOR CLINICAL CHEMISTRY, WASHINGTON, DC, vol. 43, no. 9, 1 January 1997 (1997-01-01), pages 1764-1770, XP002326863 ISSN: 0009-9147**

- TARKKINEN P ET AL: "ULTRARAPID, ULTRASENSITIVE ONE-STEP KINETIC IMMUNOASSAY FOR C-REACTIVE PROTEIN (CRP) IN WHOLE BLOOD SAMPLES: MEASUREMENT OF THE ENTIRE CRP CONCENTRATION RANGE WITH A SINGLE SAMPLE DILUTION" CLINICAL CHEMISTRY, AMERICAN ASSOCIATION FOR CLINICAL CHEMISTRY, WASHINGTON, DC, vol. 48, no. 2, 1 February 2002 (2002-02-01), pages 269-277, XP001115951 ISSN: 0009-9147
- HOLOWNIA P ET AL: "Effect of poly(ethylene glycol), tetramethylammonium hydroxide, and other surfactants on enhancing performance in a latex particle immunoassay of C-reactive protein" ANALYTICAL CHEMISTRY, AMERICAN CHEMICAL SOCIETY. COLUMBUS, US, vol. 73, no. 14, 15 July 2001 (2001-07-15), pages 3426-3431, XP002300351 ISSN: 0003-2700
- PEREZ-AMODIO S ET AL: "Effects of the ionic environment, charge, and particle surface chemistry for enhancing a latex homogeneous immunoassay of C-reactive protein." ANALYTICAL CHEMISTRY 15 JUL 2001, vol. 73, no. 14, 15 July 2001 (2001-07-15), pages 3417-3425, XP002490486 ISSN: 0003-2700
- PEULA J M ET AL: "Coadsorption of IgG and BSA onto sulfonated polystyrene latex: II. Colloidal stability and immunoreactivity" JOURNAL OF BIOMATERIALS SCIENCE. POLYMER EDITION, VSP, UTRECHT, vol. 7, no. 3, 1 January 1995 (1995-01-01), pages 241-251, XP008094781 ISSN: 0920-5063
- SERADYN INC.: "optibind polysyrene and optilink caroxylate-modified microparticles" SERADYN INC., 1 September 2005 (2005-09-01), pages 1-7, XP002490487
- BORQUE ET AL.: "Development and Validation of an Automated and Ultrasensitive Immunoturbidimetric Assay for C-Reactive Protein" CLINICAL CHEMISTRY, vol. 46, no. 11, 2000, pages 1839-1842,
- KONDO ET AL.: "Adsorption of gamma-Globulin, a Model Protein for Antibody, on Colloidal Particles" BIOTECHNOLOGY AND BIOENGINEERING, vol. 37, 1991, pages 537-543,
- GIACOMELLI ET AL.: "Adsorption of Immunoglobulin G on Core-Shell Latex Particles Precoated with Chaps" JOURNAL OF COLLOID AND INTERFACE SCIENCE, vol. 231, 2000, pages 283-288,
- CARYL GRIFFIN, JIM SUTOR, BRUCE SHULL: "Microparticle reagent optimization" 1994, SERADYN , INDIANAPOLIS, INDIANA, USA

**Description**

Technical Field

The present invention relates to a method for measuring an antigen by latex agglutination method and a reagent therefor.

Background Art

Immunoassays utilizing latex particles are applied to clinical tests for quantifying antigenic substances contained in body fluids such as serum, blood plasma and urine. Since they enable simple and rapid measurements by using automatic analyzers, they are widely used.

In recent years, applied techniques aiming at further promoting measurement performance were proposed. For example, by the reagent employing two types of latex particles having different average particle sizes on which the same antibody is immobilized in at least two different amounts (Patent Literature 1) or by the measuring method using two or more types of latex particles having different average particle sizes, sensitized with an antibody (Patent Literature 2), measurements in a wide range may be attained. By the method in which a polyclonal antibody and a monoclonal antibody are immobilized on insoluble carrier particles having a single average particle size, the same effect may be obtained (Patent Literature 3). Further, measuring methods in which particles having a small particle size coated with an antibody with a low reactivity and particles having a large particle size coated with an antibody with a high reactivity are used (Patent Literatures 4 and 5) have been proposed. Still further, Non-patent Literatures 1 and 2 disclose immunoassays by agglutination method, which use a mixed sensitized particles using two types of large and small carrier particles, wherein a monoclonal antibody having a low reaction rate is immobilized on the carrier particles having a smaller average particle size and a monoclonal antibody having a high reaction rate is immobilized on the carrier particles having a larger average particle size.

However, the methods described in these literatures have a problem in that the concentration range in which measurement may be attained is narrow, and the measurement in the high concentration region is difficult.

Patent Literature 1: Japanese Patent Publication (Kokoku) No. 63-14783
Patent Literature 2: Japanese Patent Publication No. 2588174
Patent Literature 3: Japanese Laid-open Patent Application (Kokai) No. 10-90268
Patent Literature 4: Japanese Laid-open Patent Application (Kokai) No. 11-108929
Patent Literature 5: Japanese Patent Publication (Kokai) No. 3513075
Non-patent Literature 1: Journal of Clinical Laboratory Analysis 12:137-144 (1988)
Non-patent Literature 2: Japanese Journal of Medicine and Pharmaceutical Science, 42(5):781-788,1999

Sanchez A. et al. (2002) Clin. 48, no. 5-6, pages 313-317, evaluates the analytical performance of a CRP immunoturbidometric method on a COBAS Integra 400 analyzer.

Eda S. et al (1999) Scand. J. Clin. Lab. Invest., vol. 230, pages 32-35 describes a method based on microparticle enhanced turbidometry and its use to measure C-reactive protein (CRP). The method employs a mixture of two types of microparticle reagents differing in microparticle size and reactivity of coated antibody.

EP 0 898 169 relates to a microparticle enhanced light scattering agglutination assay for determining the amount of an analyte, and a reagent for use in the assay. The assay uses a mixture of particles of strong light-scattering properties carrying at least one binding partner of high reactivity for the analyte and particles of weak light-scattering properties carrying at least one binding partner of low reactivity for the analyte.

US 2001/026945 describes reagents and the use of reagents in a microparticle light scattering agglutination assay. The reagents comprise a mixture of particles having relatively stronger light-scattering properties and particles having relatively weak light-scattering properties with respect to one another. The particles having strong light-scattering properties carry a binding partner of high reactivity with the analyte. The particles having weak light-scattering properties carry a binding partner of low reactivity with the analyte.

Eda S. et al (1998) J. Clin. Lab. Anal. vol. 12, no. 3, pages 137-144, describes a microparticle-based assay for C-reactive protein. The assay uses two different sized microparticles covalently coated with two monoclonal antibodies of different reactivity.

Katano S. (1999) Igaku to Yakugaku J. Med., vol. 42, no. 5, pages 781-788 describes a serum protein test by the fully-automated clinical chemistry analyzer COBAS Integra 400. The assay uses two different sized microparticles covalently coated with two monoclonal antibodies of different reactivity.

Benecky M. et al. (1997), Clin. Chem., vol. 43, no. 9, pages 1764-1770 describes a coupled particle light scattering method for determining the presence of multiple analytes in a medium. Multiple simultaneous assays are configured by the use of mixtures of different-size latex microparticles.

Tarkkinen P. et al. (2002), Clin. Chem., vol. 48, no. 2, pages 269-277, describes a non-competitive, kinetic CRP immunoassay that uses individual, porous microparticles covalently coated with a monoclonal capture antibody. The microparticles used were porous 60 $\mu$m acrylate microparticles.

Holownia P. et al. (2001), Anal. Chem., vol. 73, no. 14, pages 3426-3431 describes the effect of poly(ethylene glycol) and other surfactants on the performance of a latex particle immunoassay of CRP. Each assay employs a single size of latex microparticle.

Perez-Amodio S. et al. (2001), Anal. Chem. vol. 73, no. 14, pages 3417-3425 describes the effect of ionic environment, charge and particle surface chemistry on a latex particle immunoassay of CRP. Each assay employs a single size of latex microparticle.

Peula JM. Et al. (1995), J. Biomaterials Sci. vol. 7, no. 3, pages 241-251, describes the colloidal stability and immunoreactivity of latex particles covered by monomeric BSA, or an anti-CRP antibody. Incubation conditions are described that yield latex particles with high colloidal stability and reactivity.

SERADYN INC., 1 September 2005, pages 1-7 describes the manufacture and properties of polystyrene and carboxylate-modified microparticles.

Griffen C. et al. (1994) "Microparticle reagent optimization" 1994, (SERADYN INC.) is a laboratory reference manual describing the properties of microparticle regents, along with methods of their preparation and use.

Borque et al. (2000), Clin. Chem. vol. 46, no. 11, pages 1839-1842 describes th development and validation of an automated and ultrasensitive immunoturbidimetric assay for C-reactive protein. A single microparticle size was used in each assay.

Kondo et al. (1991), Biotech. and Bioeng. vol. 37, pages 537-543, describes the effects of particle surface properties on the adsorption of protein onto the particle.

Giacomelli et al. (2000), J. Colloid and Interface Sci. vol. 231, pages 283-288, describes the adsorption behaviour of a monoclonal antibody (immunoglobulin G) on latex particles having chloromethyl groups precoated with 'Chaps'.

Disclosure of the Invention

Problems Which the Invention Tries to Solve

In many of the inspection items tested in clinical immunoserological tests, the point important for making a clinical judgment is located in the low concentration range. Thus, it is apparent that the accuracy of measurement in the low concentration range largely influence the test results. Although by the techniques employing a plurality of types of latex particles having different particle sizes, a larger measurement range is attained than that attained in the methods employing a single type of particles, and measurement in the low concentration range may also be accuracy of measurement is desired. For the promotion of the accuracy of measurement, increase in the sensitivity of the measurement system is effective.

However, in the prior art, increase in the sensitivity of the measurement system accompanies reduction of the measurement range as described in Patent Literature 2.

By the methods described in Non-patent Literatures 1 and 2, measurement in the high concentration range is difficult, and the measurement range is narrow.

Accordingly, an object of the present invention is to provide a latex agglutination method by which the measurement range is extended and the measurement sensitivity in the low concentration range is promoted.

Means for Solving the Problems

The present inventors intensively studied to discover that the measurement sensitivity in the low concentration range may be increased and the measurement range in the high concentration range may be extended by using two types of large and small latex particles, that is, a mixture of latex particles having a large average particle size (large latex particles) sensitized with (carrying) an antibody in an amount at which the maximum reaction rate is attained or in an amount close thereto, and latex particles having a small average particle size (small latex particles) sensitized with the same antibody in an amount smaller than the usual amount in order to decrease the reaction rate, which mixture has the optimized average particle sizes of the large and small particles, and by optimizing the concentrations of the particles in the reaction system, thereby completing the present invention.

That is, the present invention provides a method for measuring a test antigen by latex agglutination using sensitized latex particles, the method comprising reacting sensitized latex particles suspended in a buffer with the test antigen; the sensitized latex particles being two types of large and small particles, having different average particle sizes, each of the latex particles being sensitized with the same antibody or the same antigen-binding fragment thereof, which antibody undergoes antigen-antibody reaction with the test antigen; the antibody or antigen-binding fragment thereof immobilized on the latex particles having a purity of not less than 14% by weight based on the total amount of proteins carried on the particles; the amount of the antibody or antigen-binding fragment thereof immobilized per one particle of the small particles is 0.5 to 10% by weight of the amount of the antibody or antigen binding fragment thereof immobilized per one particle of the large particles; the large latex particles having an average particle size of 0.15 to 0.29 $\mu$m; the small latex particles having an average particle size of 0.05 $\mu$m to 0.10 $\mu$m; the concentration of the large latex particles in the antigen-antibody reaction system being 0.01 to 0.04 w/v%; the concentration of the small latex particles in the antigen-antibody reaction system being 0.05 to 0.2 w/v%; and optically measuring agglutination of the sensitized latex particles. Also described herein is a reagent for measuring an antigen by latex agglutination method, comprising two types of large and small particles, having different average particle sizes, in a buffer; each of the latex particles being sensitized with the same antibody or the same antigen-binding fragment thereof, which antibody undergoes antigen-antibody reaction with the test antigen; the antibody or antigen-binding fragment thereof immobilized on the latex particles having a purity of not less than 14% by weight based on the total amount of proteins carried on the particles; the amount of the antibody or antigen-binding fragment thereof immobilized per one particle of the small particles is 0.5 to 10% by weight of the amount of the antibody or antigen-binding fragment thereof immobilized per one particle of the large particles; the large latex particles having an average particle size of 0.15 to 0.29 $\mu$m; the small latex particles having an average particle size of 0.05 $\mu$m to 0.1 0 $\mu$m.

Effects of the Invention

By the present invention, an immunoassay was established, by which measurement with high sensitivity in the low concentration range and extension of the measurement range in the high concentration range may be attained, and accuracy of measurement in the low concentration range, which is important for making a clinical judgment in immunoserological tests or the like is increased so that more accurate judgment may be attained.

Brief Description of the Drawings

Fig. 1 comparatively shows the relationships between the CRP level and the amount of change in absorbance, which were obtained using Reagent 1, 2 and 3 prepared in Example, respectively.
Fig. 2A shows the relationship between the CRP level and the amount of change in absorbance, which was obtained using Reagent 4 prepared in Example.
Fig. 2B is an enlarged view showing the low CRP concentration range in Fig. 2A.
Fig. 3A comparatively shows the relationships between the CRP level and the amount of change in absorbance, which were obtained using the reagent described herein and a reagent according to Comparative Example, respectively.
Fig. 3B is an enlarged view showing the low CRP concentration range in Fig. 3A.
Fig. 4 shows the relationship between adsorption isotherm and the maximum amount of adsorption on the adsorption 100% line.

Best Mode for Carrying Out the Invention

The latex particles used in the method of the present invention are sensitized with (i.e., carry) an antibody which undergoes antigen-antibody reaction with the antigen to be measured (hereinafter referred to as "specific antibody") or an antigen binding fragment thereof. The specific antibody may be either a polyclonal antibody or a monoclonal antibody. The particles may be sensitized with, instead of the antibody, a fragment of the antibody, having a binding ability with the corresponding antigen, such as Fab fragment or F(ab')$_2$ fragment (referred to as "antigen-binding fragment" in the present description and claims).
The purity of the antibody or the antigen-binding fragment thereof immobilized on the latex particles is not less than 14% by weight, preferably not less than 16% by weight based on the amount of proteins carried on the particles. If the purity is less than 14% by weight, sufficient measurement sensitivity in the low concentration range is not obtained. For example, a polyclonal antibody is usually prepared by injecting an antigen to an animal; collecting the blood from the animal after a prescribed time has passed; and subjecting the collected blood as a starting material to affinity chromatography using a Protein A column or the like. Thus, the polyclonal antibody contains other immunoglobulins in addition to the specific antibody which undergoes antigen-antibody reaction with the antigen. In the present invention, the percentage (purity) of the amount of the specific antibody (mg/mL) based on the amount of the total proteins (mg/mL) in the antibody solution is important, and an antibody (antibody solution) containing the specific antibody in an amount of not less than 14% by weight may preferably be used. As for a monoclonal antibody, an antibody (antibody solution) obtained by purifying ascites or culture medium containing the specific antibody as a starting material, which was obtained in growing the hybridomas producing the antibody in abdominal cavity of a mouse or in a culturing apparatus, may be used. A monoclonal antibody (antibody solution) containing the specific antibody in an amount (mg/mL) of not less than 14% by weight based on the total amount (mg/mL) of proteins may preferably be used as in the case of the polyclonal antibody.

The amount of the total proteins (mg/mL) in the antibody solution may be measured by a known method in which absorbance at a wavelength of 280nm is measured. The amount of the specific antibody may be measured by the conventional Becker's method (Experimental Methods A in Immunology, pp 164-171, 1976, published by Japanese Society for Immunology; Original article: Becker W.: Immunochemistry, 6: 539-546, 1969).

In the method of the present invention, two types of large and small particles, having different average particle sizes, are used. The latex particles having the larger average particle size (large latex particles) have an average particle size (diameter) of 0.15 to 0.29 $\mu$m, preferably 0.20 to 0.29 $\mu$m. If the average particle size of the large latex particles is within this range, a high measurement sensitivity is attained in the low concentration range. On the other hand, the average particle size (diameter) of the latex particles having the smaller average particle size (small latex particles) is 0.05 to 0.10 $\mu$m, preferably 0.06 to 0.08 $\mu$m. If the average particle size of the small latex particles is within this range, an accurate measurement in the high concentration range may be attained, so that the concentration range in which measurement may be attained is extended to the side of high concentration.

As the latex particles, those particles which are conventionally and widely used, such as, for example, those made of a polystyrene, styrene-butadiene copolymer, styrene-styrene sulfonate copolymer, may be employed.

The above-described large and small latex particles are sensitized with the same specific antibody or the same antigen-binding fragment thereof. Sensitization of the latex particles with the specific antibody or the antigen-binding fragment thereof may be carried out by a conventional well-known method. For example, sensitization may be attained by suspending the latex particles in a solution of the specific antibody or the antigen-binding fragment thereof and stirring the suspension so as to physically adsorb the specific antibody or the antigen-binding fragment thereof.

In the present invention, the amount (average) of the antibody or antigen binding fragment thereof immobilized per one particle of the small particles is 0.5 to 10% by weight of the amount (average) of the antibody or antigen-binding fragment thereof immobilized per one particle of the large particles. Since the reaction rate may be decreased by decreasing the amount of the antibody immobilized on the latex particles, the optimum amount of the antibody may be selected from the measured time course of the reaction. By so doing, the calibration curve may be extended to the side of the high concentration range, and the concentration range in which measurement may be attained is extended to the side of high concentration. The amount of the antibody or antigen-binding fragment thereof immobilized on the particles may be freely adjusted by diluting the specific antibody or antigen-binding fragment thereof with a buffer solution. The optimum amount of sensitization within the above-described range may appropriately be selected depending on the type of the antigen to be measured, purpose of measurement, measurement range, antibody titer and so on (see Test Examples below).

On the other hand, the larger the amount of the antibody adsorbed on the latex particles, the higher the reaction rate, and the larger the particle size, the higher the reaction rate. Thus, by sensitizing the large latex particles with the antibody or antigen-binding fragment thereof in an amount as large as possible, the measurement sensitivity in the low concentration range is increased. Therefore, the amount of the specific antibody or antigen-binding fragment thereof immobilized on the large latex particles is preferably as much as possible. Therefore, the amount of 85% to 120%, more preferably 95% to 120% of the maximum adsorption amount on adsorption isotherm 100% line is preferred. However, the amount of the specific antibody or antigen-binding fragment thereof may appropriately be selected depending on the type of the antigen to be measured, purpose of measurement, measurement range, antibody titer and so on. The maximum adsorption amount on adsorption isotherm 100% line may easily be determined based on the concept of adsorption isotherm well-known in the art of surface chemistry. This method is described in, for example, Microparticle Reagent Optimization, Seradyn, Inc. 1994, and will be described below in detail.

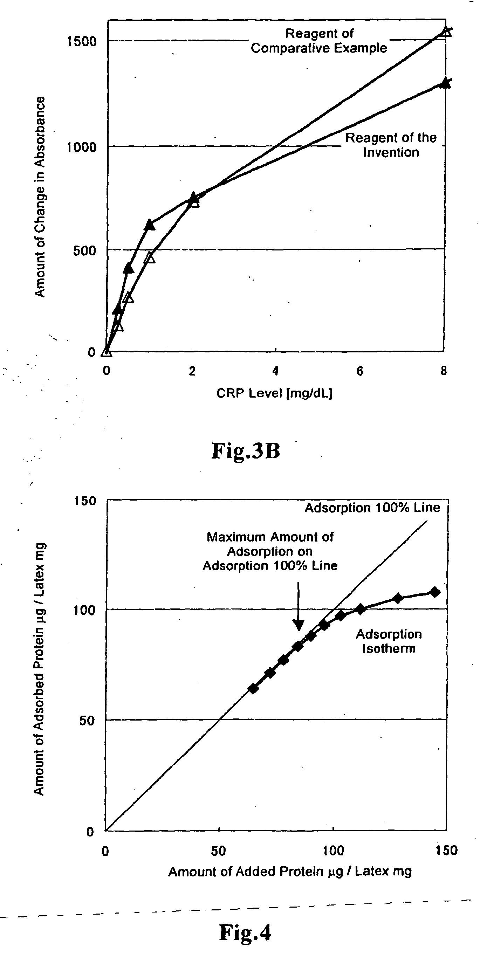

The maximum adsorption amount on the 100% line shown by adsorption isotherm (adsorption isotherm 100% line) may concretely be determined as follows: That is, the amount of protein ($\mu$g) per 1 mg of latex particles, which protein was added during the sensitization step of the latex particles, is taken along the X-axis, and the amount of the adsorbed protein ($\mu$g) per 1 mg of latex particles, which was measured after sensitization is taken along the Y-axis, thereby preparing an adsorption isotherm. Then a straight line (adsorption 100% line) of Y=X is drawn, and the maximum adsorption on the adsorption 100% line is determined. To more comprehensively explain these, a graph is shown in Fig. 4. The amount of the adsorbed protein ($\mu$g) per 1 mg of the latex particles is determined by measuring the amount of protein ($\mu$g) after filtration of the centrifugation supernatant after sensitization through a 0.1 $\mu$m filter unit (ULTRAFREE-MC, Millipore), and subtracting the thus measured amount from the amount of protein ($\mu$g) before sensitization. The adsorption ratio (%) is calculated according to the following equation:

$$\text{Adsorption Ratio (\%)} = \frac{\text{Amount of Adsorbed Protein (μg)}}{\text{Protein Amount before Sensitization (μg)}}$$

In the method of the present invention, the concentration of the large latex particles in the reaction system is 0.01 to 0.04 w/v%, and the concentration of the small latex particles in the reaction system is 0.05 to 0.2 w/v%. If the concentrations of the large and small latex particles are within these ranges, highly sensitive measurement in the low concentration range and extension of measurement range in the high concentration range may be better attained.

Latex agglutination method *per se* may be carried out in exactly the same manner as in the conventional method. That is, latex agglutination method may be carried out by mixing a test sample and a suspension of the above-described large and small sensitized latex particles in a buffer, and optically measuring the agglutination caused by the antigen-antibody reaction. The optical measurement may be carried out as follows: For example, when the latex particles sensitized with

the antibody and a test sample are mixed in a cell made of plastics or glass, if the antigenic substance to be measured exists in the test sample, the latex particles sensitized with the antibody agglutinate due to the antigen-antibody reaction. At that time, by radiating a light from the outside, which has a suitable wavelength selected from the range of 0.3 μm to 2.4 μm and measuring the change in absorbance, the target antigenic substance existing in the test sample may be measured. The change in absorbance may be measured by measuring the change in absorbance between 60 seconds and 90 seconds from the beginning of the antigen-antibody reaction. However, the measurement time is not restricted to this range, and the target antigenic substance may be measured by measuring the change in absorbance or the like during an arbitrary time period within a relatively early stage of the reaction. Since the rate of agglutination correlates with the antigen level in the test sample, the antigen level in a test sample may be determined by applying the measured agglutination rate to a calibration curve which was preliminarily prepared by subjecting some standard solutions having known antigen levels, respectively, to the reaction and by measuring the agglutination rate. The reaction temperature is not restricted, and a temperature of about 37°C is usually preferred.

The antigen which may be measured by the method of the present invention is not restricted at all, and any antigen whose corresponding antibody is able to be prepared may be measured by the method of the present invention. Examples of the antigen include proteins serving as a disease marker such as C-reactive protein (CRP), and pathogens such as bacteria and viruses, but the antigen is not restricted thereto. The test sample is also not restricted at all, and examples thereof include, but not limited to, body fluids such as serum, blood plasma, urine and throat swab; culture media; foods and beverages and extracts thereof. In cases where it is difficult to attain an accurate measurement when a body fluid or the like is applied to the measurement as it is because the antigen level is too high, needless to say, a dilution of the body fluid or the like diluted with physiological saline may be used as the test sample.

Also described herein is a reagent to be used in the above described method according to the present invention. That is, described herein is a reagent for measuring an antigen by latex agglutination method, comprising two types of large and small particles, having different average particle sizes, in a buffer; each of the latex particles being sensitized with the same antibody or the same antigen-binding fragment thereof, which antibody undergoes antigen-antibody reaction with the test antigen; the antibody or antigen-binding fragment thereof immobilized on the latex particle having a purity of not less 14% by weight based on the total amount of proteins carried on the particle; the amount of the antibody or antigen-binding fragment thereof immobilized per one particle of the small particles being 0.5 to 10% by weight of the amount of the antibody or antigen-binding fragment thereof immobilized per one particle of the large particles; the large latex particles having an average particle size of 0.15 to 0.29 μm; the small latex particles having an average particle size of 0.05 μm to 0.10 μm.

By using the small sensitized latex particles whose reaction rate is small, the method of the present invention enables to effectively capture the antigenic substance in the test sample depending on the concentration thereof, thereby enhancing the accuracy of the measurement. On the other hand, in cases where the concentration of the antigenic substance in the test sample is very small, the large sensitized latex particles preferentially capture the antigenic substance to cause agglutination, so that a small degree of reaction may be detected as a large optical change. In cases where the concentration of the antigenic substance in the test sample is high, the antibody on the large latex particles whose reaction rate is high is rapidly consumed and the large sensitized latex particles instantly agglutinate. On the other hand, the small sensitized latex particles whose reaction rate is low keep slow reaction and the agglutination reaction of the small sensitized latex particles is kept, so that the reaction may be continued while reducing the optical change. According to the method of the present invention, the measurement range is extended by these effects, and the measurement in the low concentration range may be attained with a higher sensitivity. Further, according to the present invention, by optimizing the average particle size of the large and small latex particles carrying the optimum

EP 1 801 590 B1

amount of the antibody, and by optimizing the ratio of concentrations thereof in the reaction system, extension of the measurement range and the increase in the sensitivity of the measurement in the low concentration range are especially well attained.

The method of the present invention will now be described more concretely by way of Examples thereof. However, the present invention is not restricted to the Examples below.

Example 1 Measurement of CRP

Polystyrene latex particles having a particle size of 0.28 $\mu m$ and polystyrene latex particles having a particle size of 0.06 $\mu$m are sensitized with an anti-CRP rabbit polyclonal antibody (an antibody solution in which the amount of the specific antibody (mg/mL) based on the total protein amount (mg/mL) was 17%), and the resulting latex particles were suspended in glycine buffer to prepare a reagent. That is, the reagent was prepared by mixing prescribed amounts of the antibody and the polystyrene latex particles in a buffer (MES 50mM pH 6.0, MES: 2-Morpholinoethanesulfonic acid, monohydrate); sufficiently stirring the mixture at room temperature for 60 minutes, thereby carrying out sensitization; removing supernatant after centrifugation; blocking the unsensitized regions in the surface of the polystyrene latex particles with glycine buffer containing bovine serum albumin; collecting again the polystyrene latex particles sensitized with the antibody by centrifugation; and dispersing and suspending the latex particles in glycine buffer by sufficiently conducting sonication.

One type of the polystyrene latex particles with a particle size of 0.28 $\mu$m sensitized with the antibody in an amount of 120% of the maximum adsorption amount on the adsorption 100% line shown by the adsorption isotherm; and three types of polystyrene latex particles with a particle size of 0.06 $\mu$m sensitized with the antibody in an amount of 2.29, 1.61 or 1.45% by weight of the amount of the antibody immobilized per one particle of the polystyrene latex particles with a particle size of 0.28 $\mu$m were prepared.

The amount of the immobilized antibody was concretely controlled by carrying out the sensitization after preliminarily diluting the antibody in a buffer (MES, 50mM, pH 6.0) to a concentration suited for the desired amount of the antibody to be immobilized.

Reagents Constituted by Each Type of Particles before Mixing

As examples of the reagents described herein, suspensions of the above-described particles with a particle size of 0.06 $\mu$m sensitized with the antibody in amounts of 2.29, 1.61 and 1.45% by weight, respectively, suspended in glycine buffer to a particle concentration of 0.175% (w/v) were prepared and designated Reagents 1, Reagent 2 and Reagent 3, respectively. A suspension of the antibody-carrying particles with a particle size of 0.28 $\mu$m suspended in glycine buffer to a concentration of 0.04% (w/v) was prepared and designated Reagent 4.

Reagent Constituted by Mixed Particles

A reagent as described herein was prepared by mixing the antibody-carrying particles with a particle size of 0.28 $\mu$m and the particles (the same particles as used in Reagent 2) with a particle size of 0.06 $\mu$m carrying the antibody in an amount of 1.61 % by weight in glycine buffer so as to attain particle concentrations of 0.04% (w/v) and 0.175% (w/v), respectively. As Comparative Example, a reagent prepared by the method described in Patent Literature 5 was used.

As a sample for measurement, CRP standards prepared by diluting purified human CRP antigen with normal human serum were used. The concentrations of the CRP standards were in accordance with Blood Plasma Proteins International Standards Preparations CRM470. Standards having concentrations of 0.25, 0.5, 1,2,4,8, 16, 32, 48, 67 and 100 mg/dL, respectively, were prepared. As a standard with a concentration of 0 mg/dL, physiological saline was used. Measurements were carried out using Automatic Analyzer for Biochemical Tests TBA-80FR (produced by TOSHIBA) under the following conditional parameters: That is, to 3 $\mu$L of the sample for measurement, 150 $\mu$l of glycine buffer as a first reagent, and 150 $\mu$l of the prepared reagent as a second regent were added, and the analyzer was set such that the change in absorbance ($\Delta$ABS/min) per 1 minute between the Photometry Point 21 and Photometry Point 27 after the beginning of the reaction (after adding the second reagent) was measured at a wavelength of 572 cm. $\Delta$ABS/min x 10,000 was defined as the change in absorbance.

Test Example 1

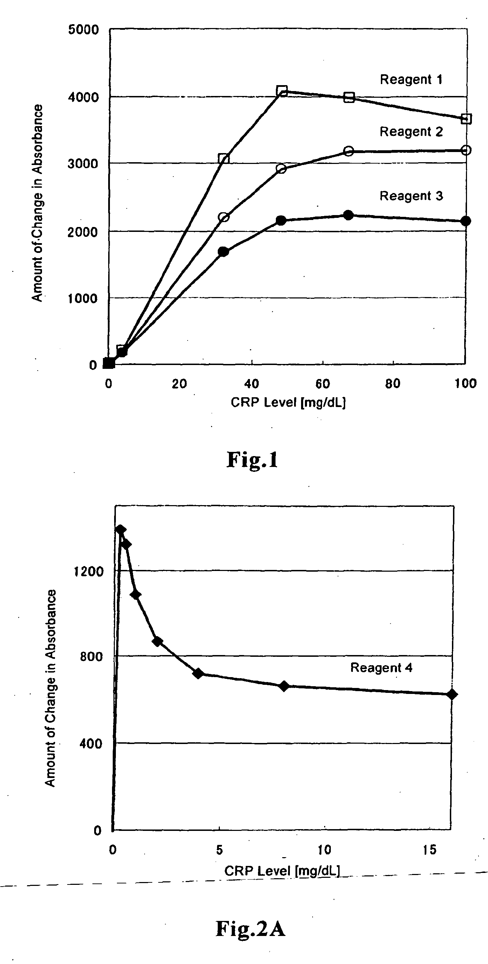

Calibration curves showing the relationships between the CRP level and the amount of change in absorbance, using Reagent 1, 2 and 3, respectively, were prepared. These are shown in Fig. 1. As shown in Fig. 1, with Reagent 1, since the reaction rate is high, the calibration curve rises up to only 48 mg/dL. With Reagent 2, since the reaction rate is lower than that with Reagent 1, the calibration curve rises up to 100 mg/dL. With Reagent 3, since the reaction rate is too low, the initial rise of the calibration curve is low. Further, since the amount of the antibody necessary for the reaction is insufficient, the calibration curve rises up to only 67 mg/dL. From these results, it can be seen that by using Reagent 2, the calibration curve rises in the high concentration range, so that accurate measurement in the high concentration range and extension of measurable concentration range to the side of the high concentration may be attained. Thus, the effect of slowing the reaction by making the amount of the antibody adsorbed to the latex particles small is evident.

Test Example 2

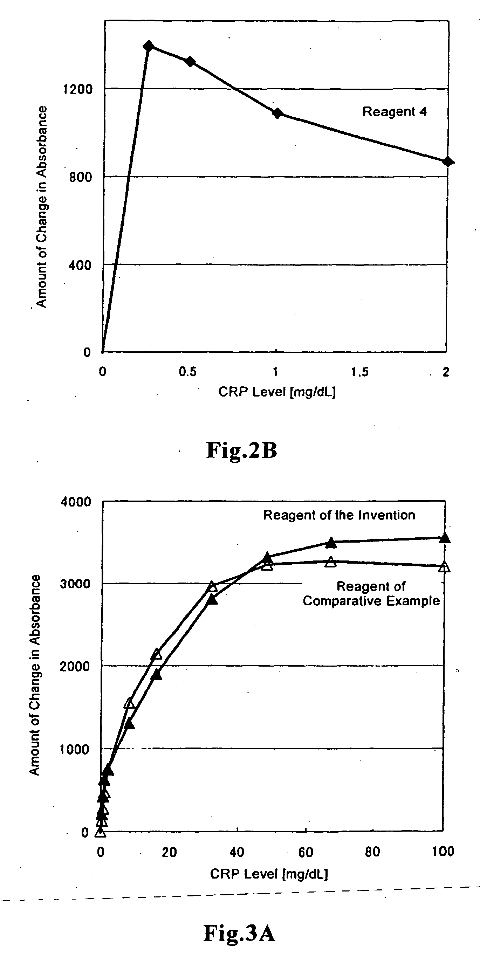

A calibration curve showing the relationship between the CRP level and the amount of change in absorbance was prepared using Reagent 4. This is shown in Figs. 2A and 2B. Fig. 2B enlargingly shows the area of the CRP level of 0 to 2 mg/dL. The calibration curve well rises in the low concentration range between 0 and 0.25 mg/dL, so that it can be seen that the measurement sensitivity is high. However, since the antibody reacts instantly, the calibration curve does rise in the high concentration range.

Test Example 3

Calibration curves showing the relationships between the CRP level and the amount of change in absorbance, using the reagent described herein and the reagent according to Comparative Example, respectively, were prepared. These are shown in Figs. 3A and 38. Fig. 3B enlargingly shows the area of the CRP level of 0 to 8 mg/dL. As shown by the results with the reagent described herein, detection with higher sensitivity than that with the reagent of Comparative Example is attained in the low concentration range between 0 and 2 mg/dL (see Fig. 3B). Further, in the high concentration range, while measurement may be attained up to only 67 mg/dL with the reagent according to Comparative Example, measurement may be attained up to 100 mg/dL with the reagent described herein (see Fig. 3A). As shown in Figs. 3A and 3B, with the reagent described herein, measurement may be attained with a higher sensitivity in the range of low CRP level and measurement may be attained up to higher concentration range than with the reagent according to Comparative Example. Further, by comparing the two calibration curves obtained with Reagent 2 shown in Fig.1 and obtained with the reagent described herein shown in Fig.3A, it can be seen that with the reagent described herein, the change in absorbance in the high concentration range of 67 to 100 mg/dL is larger than that with Reagent 2, so that it can be seen that mixing of the two types of the particles is also effective for amplifying the amount of change in absorbance. From these results, it is apparent that the method of the present invention using two types of large and small latex particles, having different average particle sizes, on which prescribed amounts of the same antibody are immobilized, respectively, that is, using large sensitized latex particles exhibiting high reaction rate and small sensitized latex particles exhibiting low reaction rate, is superior.

Industrial Availability

By the immunoassay according to the present invention, highly sensitive measurement in the low concentration range and extension of the measurement range in the large concentration range may be attained, so that the accuracy of measurement in the low concentration range, which is important for the clinical judgment in immunoserological tests and the like, is increased and more accurate judgment may be made. Therefore, the present invention is useful in immunoassays by latex agglutination method widely used in diagnoses of various diseases and the like.

**Claims**

1.  A method for measuring a test antigen by latex agglutination using sensitized latex particles, said method comprising:

    reacting sensitized latex particles suspended in a buffer with said test antigen; said sensitized latex particles being two types of large and small particles, having different average particle sizes, each of said latex particles being sensitized with the same antibody or the same antigen-binding fragment thereof, which antibody undergoes antigen-antibody reaction with said test antigen; said antibody or antigen-binding fragment thereof immobilized on said latex particles having a purity of not less than 14% by weight based on the total amount of proteins carried on said particles; the amount of said antibody or antigen-binding fragment thereof immobilized per one particle of said small particles being 0.5 to 10% by weight of the amount of said antibody or antigen-binding fragment thereof immobilized per one particle of said large particles; said large latex particles having an average particle size of 0.15 to 0.29 $\mu$m; said small latex particles having an average particle size of 0.05 $\mu$m to 0.10

μm; the concentration of said large latex particles in the antigen-antibody reaction system being 0.01 to 0.04 w/v%; the concentration of said small latex particles in the antigen-antibody reaction system being 0.05 to 0.2 w/v%; and

optically measuring agglutination of said sensitized latex particles.

**2.** The method according to claim 1, wherein said antibody or antigen-binding fragment thereof is immobilized on said large latex particle in an amount of 85% to 120% of the maximum adsorption amount on adsorption isotherm 100% line.

**Patentansprüche**

**1.** Verfahren zum Messen eines Testantigens durch Latexagglutination unter Verwendung sensibilisierter Latexpartikel, wobei das Verfahren Folgendes umfasst:

das Umsetzen von in einem Puffer suspendierten sensibilisierten Latexpartikeln mit dem Testantigen; wobei die sensibilisierten Latexpartikel zwei Arten von großen und kleinen Partikeln mit unterschiedlichen mittleren Partikelgrößen sind, wobei jedes der Latexpartikel mit dem gleichen Antikörper oder dem gleichen antigenbindenden Fragment davon sensibilisiert ist, wobei der Antikörper eine Antigen-Antikörper-Reaktion mit dem Testantigen durchläuft; wobei der Antikörper oder das antigenbindende Fragment davon, auf den Latexpartikeln immobilisiert, eine Reinheit von nicht weniger als 14 Gew.-%, bezogen auf die Gesamtmenge an auf diesen Partikeln getragenen Proteinen, aufweist; wobei die Menge des pro Partikel der kleinen Partikel immobilisierten Antikörpers oder antigenbindenden Fragments davon 0,5 bis 10 Gew.-% der Menge des pro Partikel der großen Partikel immobilisierten Antikörpers oder antigenbindenden Fragments davon beträgt; wobei die großen Latexpartikel eine mittlere Partikelgröße von 0,15 bis 0,29 μm aufweisen; wobei die kleinen Latexpartikel eine mittlere Partikelgröße von 0,05 bis 0,10 μm aufweisen; wobei die Konzentration der großen Latexpartikel im Antigen-Antikörper-Reaktionssystem 0,01 bis 0,04 % (Gew./Vol.) beträgt; wobei die Konzentration der kleinen Latexpartikel im Antigen-Antikörper-Reaktionssystem 0,05 bis 0,2 % (Gew./Vol.) beträgt; und das optische Messen der Agglutination der sensibilisierten Latexpartikel.

**2.** Verfahren nach Anspruch 1, worin der Antikörper oder das antigenbindende Fragment davon auf dem großen Latexpartikel in einer Menge von 85 % bis 120 % der maximalen Adsorptionsmenge auf der 100-%-Linie der Adsorptionsisotherme immobilisiert ist.

**Revendications**

**1.** Procédé pour mesurer un antigène test par agglutination de latex en utilisant des particules de latex sensibilisées, ledit procédé comprenant:

faire réagir les particules de latex sensibilisées suspendues dans un tampon avec ledit antigène test; lesdites particules de latex sensibilisées étant deux types de particules grandes et petites, ayant des tailles de particule moyennes différentes, chacune desdites particules de latex étant sensibilisées avec le même anticorps ou le même fragment de liaison d'antigène de celui-ci, ledit anticorps subit une réaction antigène-anticorps avec ledit antigène test; ledit anticorps ou fragment de liaison d'antigène de celui-ci immobilisé sur lesdites particules de latex ayant une pureté non inférieure à 14% en poids basée sur la quantité totale de protéines supportées sur lesdites particules; la quantité dudit anticorps ou fragment de liaison d'antigène de celui-ci immobilisée par particule desdites petites particules étant de 0,5 à 10% en poids de la quantité dudit anticorps ou fragment de liaison d'antigène de celui-ci immobilisé par particule desdites grandes particules; lesdites grandes particules de latex ayant une taille de particule moyenne de 0,15 à 0,29 μm; lesdites petites particules de latex ayant une taille de particule moyenne de 0,05μm à 0,10μm; la concentration desdites grandes particules de latex dans le système de réaction antigène-anticorps étant de 0,01 à 0,04% en p/v; la concentration desdites petites particules de latex dans le système de réaction d'antigène-anticorps étant de 0,05 à 0,2% en p/v; et mesurer optiquement l'agglutination desdites particules de latex sensibilisées.

**2.** Procédé selon la revendication 1, dans lequel ledit anticorps ou fragment de liaison d'antigène de celui-ci est immobilisé sur ladite grande particule de latex en une quantité de 85% à 120% de la quantité d'absorption maximale sur une ligne d'isotherme d'absorption de 100%.

**Fig.1**

**Fig.2A**

**Fig.2B**

**Fig.3A**

**Fig.3B**

**Fig.4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 63014783 B
- JP 2588174 B
- JP 10090268 A
- JP 11108929 A
- JP 3513075 A
- EP 0898169 A
- US 2001026945 A

### Non-patent literature cited in the description

- *Journal of Clinical Laboratory Analysis,* 1988, vol. 12, 137-144
- *Japanese Journal of Medicine and Pharmaceutical Science,* 1999, vol. 42 (5), 781-788
- **Sanchez A. et al.** *Clin.,* 2002, vol. 48 (5-6), 313-317
- **Eda S. et al.** *Scand. J. Clin. Lab. Invest.,* 1999, vol. 230, 32-35
- **Eda S. et al.** *J. Clin. Lab. Anal.,* 1998, vol. 12 (3), 137-144
- **Katano S.** *Igaku to Yakugaku J. Med.,* 1999, vol. 42 (5), 781-788
- **Benecky M. et al.** *Clin. Chem.,* 1997, vol. 43 (9), 1764-1770
- **Tarkkinen P. et al.** *Clin. Chem.,* 2002, vol. 48 (2), 269-277
- **Holownia P. et al.** *Anal. Chem.,* 2001, vol. 73 (14), 3426-3431
- **Perez-Amodio S. et al.** *Anal. Chem.,* 2001, vol. 73 (14), 3417-3425
- **Peula JM. et al.** *J. Biomaterials Sci.,* 1995, vol. 7 (3), 241-251
- Microparticle reagent optimization. **Griffen C. et al.** Microparticle reagent optimization. SERADYN INC, 1994
- **Borque et al.** *Clin. Chem.,* 2000, vol. 46 (11), 1839-1842
- **Kondo et al.** *Biotech. and Bioeng.,* 1991, vol. 37, 537-543
- **Giacomelli et al.** *J. Colloid and Interface Sci.,* 2000, vol. 231, 283-288
- Experimental Methods A in Immunology. Japanese Society for Immunology, 1976, 164-171
- **Becker W.** *Immunochemistry,* 1969, vol. 6, 539-546
- Microparticle Reagent Optimization. Seradyn, Inc, 1994